# EUROPEAN PATENT SPECIFICATION

(11) **EP 4 302 692 B1**
(45) Date of publication and mention of the grant of the patent: **15.07.2026**
(21) Application number: 22763121.5
(22) Date of filing: 25.02.2022
(51) Int. Cl.: A61B 5/256, A61B 5/291

(54) **BRAIN WAVE DETECTION ELECTRODE AND BRAIN WAVE MEASUREMENT DEVICE**
ELEKTRODE ZUR ERKENNUNG VON GEHIRNWELLEN UND VORRICHTUNG ZUR MESSUNG VON GEHIRNWELLEN
ÉLECTRODE DE DÉTECTION D'ONDE CÉRÉBRALE ET DISPOSITIF DE MESURE D'ONDE CÉRÉBRALE

(30) Priority: 02.03.2021 JP 2021032537
(43) Date of publication of application: 10.01.2024
(73) Proprietor: Sumitomo Bakelite Co., Ltd., Shinagawa-ku Tokyo 140-0002 (JP)
(72) Inventor: SAWADA, Masashi, Tokyo 140-0002 (JP); KITAZOE, Katsuma, Tokyo 140-0002 (JP)
(74) Representative: Ter Meer Steinmeister & Partner
(86) International application number: PCT/JP2022/007834
(87) International publication number: WO 2022/186065

(56) References cited:
- WO-A1-2019/094609
- WO-A1-2020/085035
- WO-A1-2020/213626
- WO-A1-2023/048065
- JP-A- 2002 165 770
- JP-A- 2016 163 688
- JP-A- 2016 163 688
- JP-A- 2022 033 419
- JP-U- 3 209 880
- JP-U- 3 209 880
- US-A1- 2018 125 386
- US-A1- 2018 125 386

## Description

### TECHNICAL FIELD

The present invention relates to a brain wave detection electrode and a brain wave measurement device.

### BACKGROUND ART

Various developments have been made with regard to brain wave detection electrodes. As technologies of this kind, for example, the technologies disclosed in Patent Document 1 and Patent Document 2 are known. Brain wave detection electrodes are also known from WO 2020/213626 A1, US 2018/125386 A1, JP 3 209880 U and JP 2016 163688 A.

The bioelectrode (brain wave detection electrode) of Patent Document 1 is provided with an electrode member of conductive rubber having a pillar shape with rounded ends.

The electrode for brain wave measurement (brain wave detection electrode) disclosed in Patent Document 2 is provided with a base portion, a protruding portion formed of rubber provided to protrude from the base portion, and a contact portion formed of metal which is provided at the tip of the protruding portion, which is electrically connected to the outside of the electrode for brain wave measurement, and which contacts the scalp during brain wave measurement.

### RELATED DOCUMENT

### PATENT DOCUMENT

[Patent Document 1] International Publication No. WO2018/230445
[Patent Document 2] Japanese Patent No. 5842198

### SUMMARY OF THE INVENTION

### TECHNICAL PROBLEM

In general, the larger the contact area is between the electrode part of an electrode for brain wave measurement and the scalp, the greater the extent to which the ability to acquire clean brain waves is improved. In the technologies of Patent Documents 1 and 2, the structure is such that the electrode part is provided at the tip portion of a protruding portion (protruding part) having a cylindrical shape or a conical shape (also including shapes in which the tip portion is cut off). In this type of structure, there may be cases where deformation such as buckling occurs as the electrode is pressed against the head, or where slippage occurs on the electrode-scalp contact surface, such that the hair interferes with the electrode during the process, and there is a demand for new technology able to eliminate the above.

The present invention was made in consideration of the above circumstances and has an object of providing a technique with which, in an electrode for brain wave measurement in which an electrode part is provided at the tip portion of a protruding portion (protruding part), it is possible to suppress deformation when the protruding portion is pressed against the scalp and to appropriately maintain a state of contact between the electrode and the scalp even in a case where deformation occurs.

### SOLUTION TO PROBLEM

According to the present invention, there is provided a brain wave detection electrode including a pillar-shaped base part, a plurality of polygonal pyramid-shaped protruding parts that are elastic bodies and provided perpendicularly to one end face of the base part, and electrode parts provided on tip portions of the protruding parts, in which H/D, which is a ratio As of a width D of a bottom surface of the polygonal pyramid shapes to a height H of the polygonal pyramid shapes, is equal to or more than 1.2 and equal to or less than 2.0; wherein the polygonal pyramid shape is a square pyramid or a hexagonal pyramid, wherein, in the plurality of the protruding parts, polygons of bottom surfaces of adjacent protruding parts are in contact with each other, wherein a shortest distance L between vertices of adjacent protruding parts is equal to or more than 2 mm and equal to or less than 5 mm, and wherein a type A durometer hardness E measured at 37°C and in accordance with JIS K 6253 (1997) of the elastic bodies is equal to or more than 15 and equal to or less than 55.

According to the present invention, there is provided a brain wave measurement device provided with the brain wave detection electrode described above.

### ADVANTAGEOUS EFFECTS OF INVENTION

According to the present invention, it is possible to provide a technique with which, in an electrode for brain wave measurement in which an electrode part is provided at the tip portion of a protruding portion, when the protruding portion is pressed against the scalp, it is possible to suppress deformation and to appropriately maintain a state of contact between the electrode and the scalp even in a case where deformation occurs.

### BRIEF DESCRIPTION OF THE DRAWINGS

Fig. 1 is a diagram schematically showing a brain wave measurement device according to an embodiment, in a state of being mounted on a human head.
Fig. 2 is a perspective view of a frame, according to an embodiment.
Fig. 3 is a front view of a brain wave electrode unit, according to an embodiment.
Fig. 4 is a plan view of a brain wave detection electrode, according to an embodiment.
Fig. 5 is a diagram in which two protrusions are viewed from the side (circumferential side), according to an embodiment.
Fig. 6 is a plan view of a brain wave detection electrode in which the protrusions are hexagonal pyramids, according to an embodiment.
Figs. 7A and 7B are diagrams which simulate protrusions in a deformed state, according to an embodiment.
Figs. 8A and 8B are diagrams which simulate protrusions of a type of the related art in a deformed state, according to an embodiment.

### DESCRIPTION OF EMBODIMENTS

### <Summary>

A description will be given below of embodiments of the present invention using the drawings.

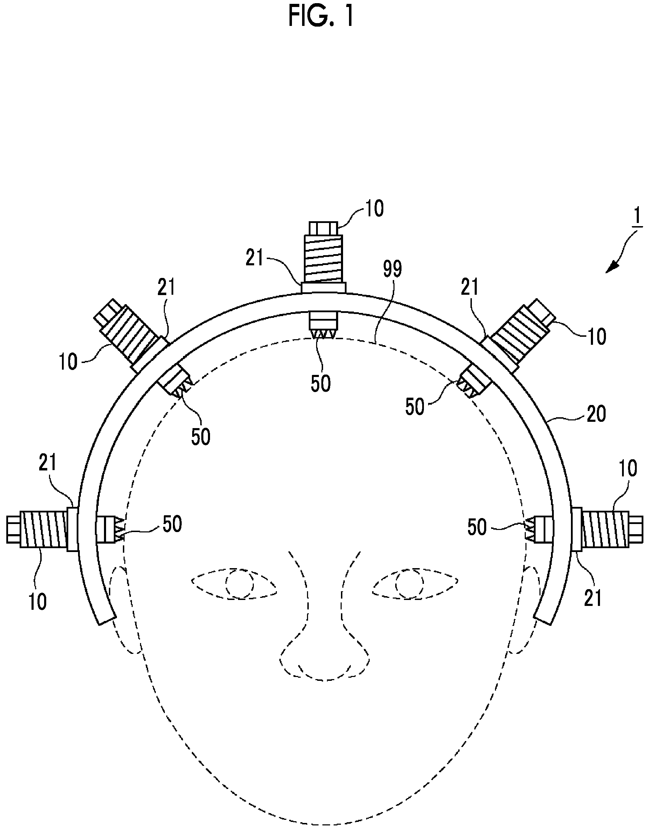

Fig. 1 is a diagram schematically showing a brain wave measurement device 1 in a state of being mounted on a head 99 of a person.

The brain wave measurement device 1 is mounted on the head 99 of a person, detects brain waves as fluctuations in potential from a living body, and outputs the detected brain waves to a brain wave display device (not shown). The brain wave display device acquires the brain waves detected by the brain wave measurement device 1 and performs well-known brain wave analysis processes such as displaying the above on a monitor or storing data.

### <Structure of Brain Wave Measurement Device 1>

As shown in Fig. 1, the brain wave measurement device 1 has a plurality of brain wave electrode units 10 and a frame 20. In the present embodiment, the brain wave electrode units 10 are provided for five channels (five units).

### <Structure of Frame 20>

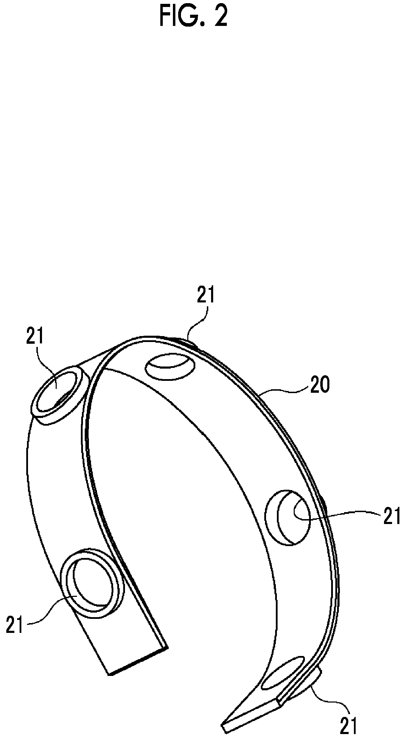

Fig. 2 shows a perspective view of the frame 20. The frame 20 is formed of a hard member of, for example, polyamide resin or the like, in a band shape which is curved to conform to the shape of the human head 99.

The frame 20 is provided with electrode unit mounting sections 21 at five locations as openings for mounting the brain wave electrode units 10. The positions of the electrode unit mounting sections 21 (that is, the mounting positions of the brain wave electrode units 10) correspond to the positions T3, C3, Cz, C4, and T4 in the International 10-20 electrode placement method.

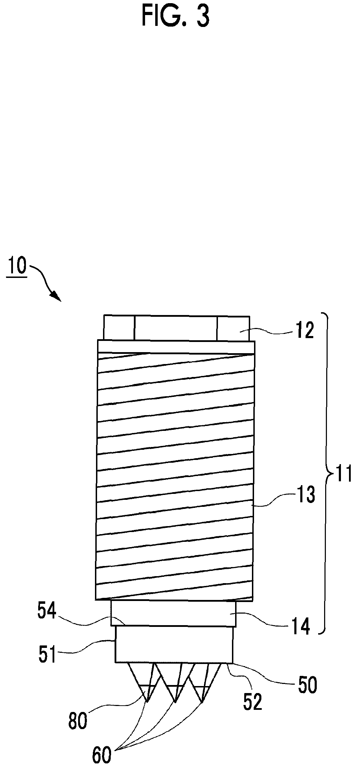

The inner circumferential surfaces of the electrode unit mounting sections 21 are threaded and the brain wave electrode units 10 are screwed in using a threaded portion 13 (refer to Fig. 3) of a body 11 thereof. By adjusting the screwing-in amount of the brain wave electrode units 10, the amount of protrusion toward the head 99 side is adjusted and the contact amount and contact pressure on the head 99 (scalp) are controlled. In addition, the operation of screwing in the brain wave electrode units 10 also parts the hair.

### <Structure of Brain Wave Electrode Unit 10>

Fig. 3 shows a front view of the brain wave electrode unit 10. The brain wave electrode unit 10 has the body 11 having an approximately cylindrical shape and a brain wave detection electrode 50 provided at one end side thereof (the lower side in the figure).

The body 11 has a signal extraction unit 12, the threaded portion 13, and an electrode fixing portion 14, which are integrated.

The threaded portion 13 has a cylindrical shape which is threaded on the side surface thereof. The signal extraction unit 12 is provided at one end (the upper side in the figure) of the threaded portion 13. The signal extraction unit 12 is provided with a signal output terminal and is manipulated by an operator using a predetermined jig as necessary when screwing the brain wave electrode unit 10 into the frame 20. At the other end of the threaded portion 13 (the lower side in the figure), there is the electrode fixing portion 14 having a cylindrical shape having a smaller diameter than the threaded portion 13. The brain wave detection electrode 50 is mounted on the electrode fixing portion 14.

### <Structure of Brain Wave Detection Electrode 50>

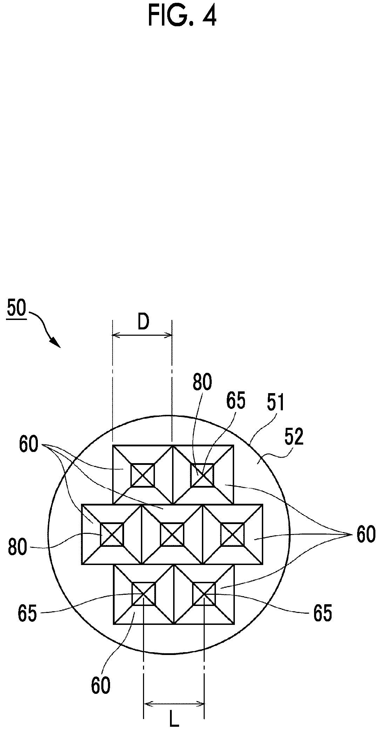

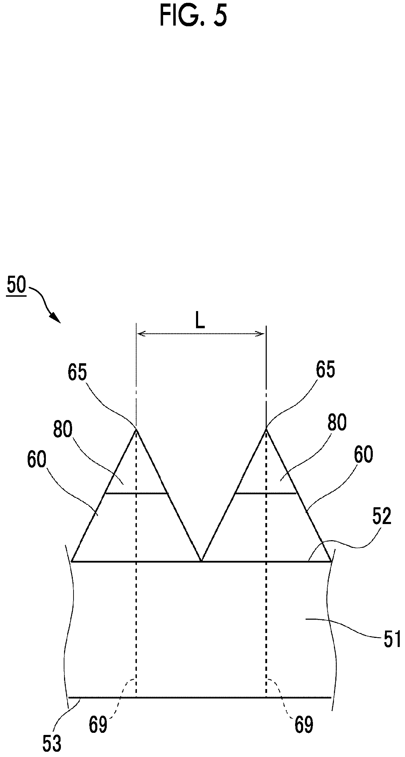

Fig. 4 is a plan view of protrusions 60 of the brain wave detection electrode 50, viewed from the tip side (the bottom side in the figure) of the protrusions 60. Fig. 5 is a diagram in which two of the protrusions 60 are viewed from the side (circumferential side).

The brain wave detection electrode 50 has a basal part 51, the protrusions 60, and electrode parts 80. The basal part 51 and the protrusions 60 are integrally provided by a rubber-like elastic body. A description will be given below of the specific materials of the elastic body. The basal part 51 and the protrusions 60 are not limited to an integrally provided configuration, but may also be configured to be provided separately and assembled using an adhesive or a fitting structure.

In a case of a rubber-like elastic body in which a hardness E of the basal part 51 and the protrusions 60 is equal to or more than 15 and equal to or less than 55, when the protrusions 60 are pressed against the head 99 of the subject, it is possible to suppress inappropriate deformation such as buckling and to appropriately maintain the contact pressure as well as to suppress the pressing pressure from being excessively strong and causing discomfort to the subject.

The basal part 51 has an approximately cylindrical shape and has a circular protrusion formation surface 52 at one end and a circular mounting surface 53 at the other end. The mounting surface 53 is mounted on the electrode fixing portion 14 of the body 11 using adhesive or the like. As the fixing structure of the mounting surface 53 and the electrode fixing portion 14, a fitting structure employing an uneven shape may be used.

The outer diameter of the brain wave detection electrode 50, that is, the diameter of the protrusion formation surface 52, is, for example, equal to or more than 10 mm and equal to or less than 30 mm.

### <Structure of Protrusion 60>

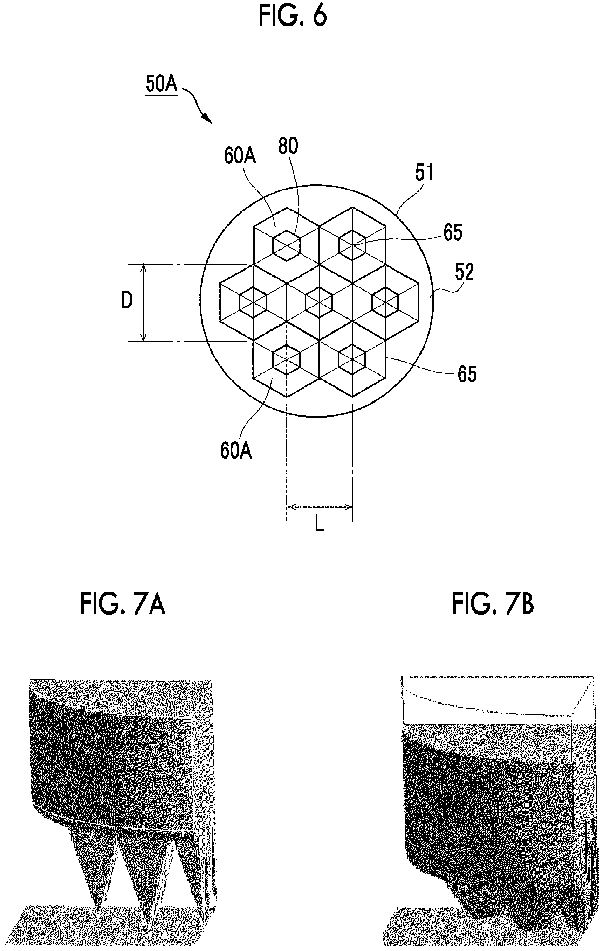

A plurality of the protrusions 60 are perpendicularly provided and arranged in alignment on the protrusion formation surface 52. In the present embodiment, as shown in Fig. 4, seven of the square pyramid protrusions 60 are provided extending integrally from the protrusion formation surface 52. Fig. 6 shows a brain wave detection electrode 50A in which protrusions 60A are hexagonal pyramids.

The protrusions 60 of the present embodiment are polygonal pyramid shapes and the bottom surfaces thereof are the boundary with the protrusion formation surface 52. The vertex of the polygonal pyramid shape is a tip portion 65 of the protrusion 60. In the present embodiment, as shown in Fig. 4, the protrusion 60 is a square pyramid, more specifically, a square cone having a square bottom surface in which a vertical line from the tip portion 65, which is the apex, to the bottom surface (that is, the protrusion formation surface 52) passes through the center of gravity of the bottom surface, forming a so-called "pyramid shape". In particular, in the case of a polygonal pyramid shape which is a square pyramid, it is easy to design the arrangement of the plurality of the protrusions 60 and to predict the state of deformation when pressed against the head 99. That is, it is possible to perform brain wave measurement in a stable manner.

The polygons, which are the bottom surfaces of the adjacent protrusions 60, are in contact with each other and are arranged without gaps therebetween. The entire arrangement of the protrusions 60 is preferably rotationally symmetrical. Due to this, when the brain wave electrode units 10 having the brain wave detection electrodes 50 are mounted on the frame 20, it is possible to appropriately adjust the contact state with the head 99, regardless of the mounting state (that is, the rotational position in the screw fitting).

The number N of the protrusions 60 is equal to or more than 4 and equal to or less than 30. The lower limit is preferably 5 or more and more preferably 7 or more. The upper limit is preferably 25 or less, more preferably 20 or less, and even more preferably 12 or less. In the present embodiment, the number N of the protrusions 60 is 7. By setting the number N of the protrusions 60 to the range described above, it is possible to appropriately adjust the contact state with the head 99. In a case where the number N of the protrusions 60 is excessively small, fine adjustment becomes difficult when the brain wave electrode units 10 mounted on the frame 20 are rotated to adjust the contact state with the head 99. On the other hand, in a case where the number N of the protrusions 60 is excessively large, the protrusions 60 become thin and deformation such as buckling increases when the protrusions 60 are pressed against the head 99, making appropriate brain wave measurement difficult. In addition, in a case where the number N of the protrusions 60 is excessively large, the force of pressing against the head 99 is felt to be strong and may be felt to be painful within a short time, or the contact feeling may be felt strongly. From this viewpoint, setting the above range makes it possible to stabilize the brain wave measurement.

The width D of the protrusions 60 (that is, the width of the bottom surface of the polygonal pyramid shape) is, for example, equal to or more than 2 mm and equal to or less than 5 **mm.** The lower limit is preferably 2.5 mm or more and more preferably 2.7 mm or more. The upper limit is preferably 4.5 mm or less and more preferably 4.0 mm or less. By setting the width D of the protrusions 60 in such a range, it is possible to arrange an appropriate quantity of the protrusions 60 on the protrusion formation surface 52.

The height H of the protrusions 60 is equal to or more than 0.5 mm and equal to or less than 20 mm. The lower limit is preferably 2 mm or more and more preferably 4 mm or more. The upper limit is preferably 15 mm or less and more preferably 10 mm or less. By setting the height H of the protrusion 60 in such a range, when the brain wave detection electrode 50 is pressed against the head 99, it is possible to prevent deformation such as buckling of the protrusion 60 even when the pressing force is small and to realize an appropriate contact state.

The ratio As = H/D of the width D of the bottom surfaces of the polygonal pyramid shapes presented by the protrusions 60 and the height H of the protrusions 60 is equal to or more than 1.2 and equal to or less than 2.0. The lower limit is preferably 1.3 or more and more preferably 1.35 or more. The upper limit is preferably 1.8 or less and more preferably 1.4 or less. By setting the ratio As with the height H of the protrusions 60 to such a range, when the brain wave detection electrode 50 is pressed against the head 99, it is possible to prevent deformation such as buckling of the protrusions 60 even when the pressing force is small and to realize an appropriate contact state. In particular, by setting the width D and the height H to the ranges described above and then setting the ratio As thereof to the range described above, it is possible to realize a suitable contact state.

A shortest distance L between the tip portions 65 of the adjacent protrusions 60 is equal to or more than 2 mm and equal to or less than 5 mm. The lower limit is preferably 2.5 mm or more and more preferably 2.7 mm or more. The upper limit is preferably 4.5 mm or less and more preferably 4.0 mm or less. By setting the shortest distance L between the tip portions 65 provided on the electrode parts 80 to such a range, it is possible to carry out the brain wave measurement by bringing the electrode parts 80 into contact at the optimum contact positions. In addition, it is possible to part the hair smoothly and to prevent hair from getting between the electrode parts 80 and the scalp.

### <Structure of Signal Line 69>

Inside the protrusions 60, conductive signal lines 69 (shown as dashed lines in Fig. 5) that connect to the electrode parts 80 are provided. For the signal lines 69, it is possible to adopt various arrangement structures as long as the form thereof carries out conduction inside the protrusions 60. For example, the tip of the signal line 69 may have any of a structure protruding from a predetermined region (an electrode formation surface) from the tip portion 65 of the protrusion 60 toward the lower side, a structure formed on approximately the same plane, or a buried structure. A protruding structure may be used from the viewpoint of connection stability with the electrode parts 80. The protruding member of the tip of the signal line 69 is partially or entirely covered by the electrode part 80.

For the protruding structure of the tip of the signal line 69, it is possible to adopt a structure without folding, with folding, or wrapping around the surface of the tip of the protrusion 60. In addition, the extension direction of the signal line 69 is not particularly limited and may be inclined with respect to the vertical line without coinciding with the vertical line extending from the protrusion formation surface 52.

### <Material of Brain Wave Detection Electrode 50>

A description will be given of the material of the brain wave detection electrode 50. The brain wave detection electrode 50 (the basal part 51 and the protrusions 60) is a rubber-like elastic body as described above. The rubber-like elastic body is specifically rubber or a thermoplastic elastomer (also referred to simply as "elastomer (TPE)"). As rubber, there is, for example, silicone rubber. As thermoplastic elastomers, there are, for example, styrene-based TPEs (TPS), olefin-based TPEs (TPO), vinyl chloride-based TPEs (TPVC), urethane-based TPEs (TPU), ester-based TPEs (TPEE), amide-based TPEs (TPAE), and the like.

In a case where the brain wave detection electrode 50 is silicone rubber, when the type A durometer hardness on the surface of the brain wave detection electrode 50 (the protrusions 60 and the basal part 51) measured at 37°C and in accordance with JIS K 6253 (1997) is defined as the rubber hardness E, the rubber hardness E is equal to or more than 15 and equal to or less than 55 and more preferably equal to or more than 25 and equal to or less than 30. The lower limit is preferably 26 or more and more preferably 27 or more. The upper limit is preferably 29 or less and more preferably 28 or less.

Here, a description will be given of the silicone rubber-based curable compositions described above.

It is possible for the silicone rubber described above to be formed of a cured product of a silicone rubber-based curable composition. The curing step of the silicone rubber-based curable resin composition is performed, for example, by heating at 100°C to 250°C for 1 to 30 minutes (primary curing) followed by post-baking at 100°C to 200°C for 1 to 4 hours (secondary curing).

Insulating silicone rubber is silicone rubber not including a conductive filler, while conductive silicone rubber is silicone rubber including a conductive filler.

It is possible for the silicone rubber-based curable composition according to the present embodiment to include a vinyl group-containing organopolysiloxane (A). The vinyl group-containing organopolysiloxane (A) is a polymer that is the main component of the silicone rubber-based curable composition of the present embodiment.

The insulating silicone rubber-based curable composition and the conductive silicone rubber-based curable composition may include vinyl group-containing linear organopolysiloxanes of the same type. Vinyl group-containing linear organopolysiloxanes of the same type may differ in terms of the amount of vinyl groups in the molecule, the molecular weight distribution, or the addition amount, as long as at least the functional group includes the same vinyl group and has a linear form.

The insulating silicone rubber-based curable composition and the conductive silicone rubber-based curable composition may further include vinyl group-containing organopolysiloxanes that differ from each other.

It is possible for the vinyl group-containing organopolysiloxane (A) described above to include a vinyl group-containing linear organopolysiloxane (A1) having a linear chain structure.

The vinyl group-containing linear organopolysiloxane (A1) described above has a linear chain structure and contains vinyl groups and these vinyl groups act as cross-linking points during curing.

The content of the vinyl groups in the vinyl group-containing linear organopolysiloxane (A1) is not particularly limited, but, for example, it is preferable to have two or more vinyl groups in the molecule and for the content to be 15 mol% or less and more preferably 0.01 mol% to 12 mol%. Due to this, the amount of vinyl groups in the vinyl group-containing linear organopolysiloxane (A1) is optimized and it is possible to reliably form a network with each component described below. In the present embodiment, "to" has a meaning including the numerical values at both ends.

In the present specification, the vinyl group content is the mol% of the vinyl group-containing siloxane units when the total of the units forming the vinyl group-containing linear organopolysiloxane (A1) is set at 100 mol%. However, one vinyl group-containing siloxane unit is considered to be one vinyl group.

In addition, the degree of polymerization of the vinyl group-containing linear organopolysiloxane (A1) is not particularly limited, but is, for example, preferably in a range of approximately 1000 to 10000 and more preferably approximately 2000 to 5000. It is possible to determine the degree of polymerization, for example, as the number-average degree of polymerization (or number-average molecular weight) in polystyrene terms in gel permeation chromatography (GPC) in which chloroform is the expansion solvent.

Furthermore, the specific gravity of the vinyl group-containing linear organopolysiloxane (A1) is not particularly limited, but is preferably in a range of approximately 0.9 to 1.1.

As the vinyl group-containing linear organopolysiloxane (A1), by using an example having a degree of polymerization and specific gravity within the above range, it is possible to improve the heat resistance, flame resistance, chemical stability, and the like of the obtained silicone rubber.

As the vinyl group-containing linear organopolysiloxane (A1), it is particularly preferable to have the structure represented by Formula (1).

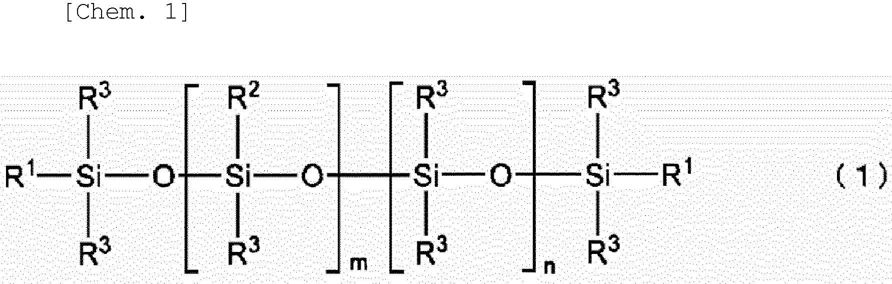

In Formula (1), R¹ is a substituted or unsubstituted alkyl group, alkenyl group, aryl group having 1 to 10 carbon atoms, or a hydrocarbon group combining the above. Examples of alkyl groups having 1 to 10 carbon atoms include methyl groups, ethyl groups, propyl groups, and the like, among which methyl groups are preferable. Examples of alkenyl groups having 1 to 10 carbon atoms include vinyl groups, allyl groups, butenyl groups, and the like, among which vinyl groups are preferable. Aryl groups having 1 to 10 carbon atoms include phenyl groups and the like.

In addition, R² is a substituted or unsubstituted alkyl group, alkenyl group, or aryl group having 1 to 10 carbon atoms or a hydrocarbon group combining the above. Examples of alkyl groups having 1 to 10 carbon atoms include methyl groups, ethyl groups, propyl groups, and the like, among which methyl groups are preferable. Examples of alkenyl groups having 1 to 10 carbon atoms include vinyl groups, allyl groups, and butenyl groups. Examples of aryl groups having 1 to 10 carbon atoms include phenyl groups.

In addition, R³ is a substituted or unsubstituted alkyl group having 1 to 8 carbon atoms, an aryl group, or a hydrocarbon group combining the above. Examples of alkyl groups having 1 to 8 carbon atoms include methyl groups, ethyl groups, propyl groups, and the like, among which methyl groups are preferable. Examples of aryl groups having 1 to 8 carbon atoms include phenyl groups.

Furthermore, examples of substituents of R¹ and R² in Formula (1) include methyl groups, vinyl groups, and the like and examples of substituents of R³ include methyl groups and the like.

In Formula (1), the plurality of R¹'s are independent of each other and may be different from each other or may be the same. Furthermore, the same also applies to R² and R³.

Furthermore, m and n are the number of repeating units forming the vinyl group-containing linear organopolysiloxane (A1) represented by Formula (1), in which m is an integer of 0 to 2000 and n is an integer of 1000 to 10000. m is preferably 0 to 1000 and n is preferably 2000 to 5000.

In addition, the specific structure of the vinyl group-containing linear organopolysiloxane (A1) represented by Formula (1) is, for example, represented by Formula (1-1).

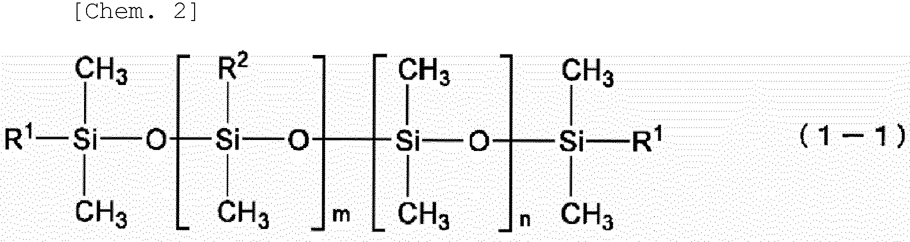

In Formula (1-1), R¹ and R² are each independently a methyl group or a vinyl group and at least one is a vinyl group.

Furthermore, as the vinyl group-containing linear organopolysiloxane (A1), an example containing a first vinyl group-containing linear organopolysiloxane (A1-1) having two or more vinyl groups in the molecule and having a vinyl group content of 0.4 mol% or less and a second vinyl group-containing linear organopolysiloxane (A1-2) having a vinyl group content of 0.5 mol% to 15 mol% is preferable. By combining a first vinyl group-containing linear organopolysiloxane (A1-1) having a typical vinyl group content and a second vinyl group-containing linear organopolysiloxane (A1-2) having a high vinyl group content as the raw rubber material for silicone rubber, it is possible to cause the vinyl groups to be unevenly distributed and more effectively form variations in the cross-linking density in the cross-linked network of the silicone rubber. As a result, it is possible to more effectively increase the tear strength of the silicone rubber.

Specifically, as the vinyl group-containing linear organopolysiloxane (A1), for example, in Formula (1-1), it is preferable to use a first vinyl group-containing linear organopolysiloxane (A1-1) having two or more units in the molecule of a unit in which R¹ is a vinyl group and/or a unit in which R² is a vinyl group and including 0.4 mol% or less thereof and a second vinyl group-containing linear organopolysiloxane (A1-2) including 0.5 mol% to 15 mol% of a unit in which R¹ which is a vinyl group and/or a unit in which R² is a vinyl group.

In addition, the first vinyl group-containing linear organopolysiloxane (A1-1) preferably has a vinyl group content of 0.01 mol% to 0.2 mol%. In addition, the second vinyl group-containing linear organopolysiloxane (A1-2) preferably has a vinyl group content of 0.8 mol% to 12 mol%.

Furthermore, in a case where the first vinyl group-containing linear organopolysiloxane (A1-1) and the second vinyl group-containing linear organopolysiloxane (A1-2) are combined and blended, the ratio of (A1-1) and (A1-2) is not particularly limited but, for example, (A1-1): (A1-2) is preferably 50:50 to 95:5 by weight ratio and more preferably 80:20 to 90:10.

The first and second vinyl group-containing linear organopolysiloxanes (A1-1) and (A1-2) may each be used as one type only or in a combination of two or more types.

In addition, the vinyl group-containing organopolysiloxane (A) may also include a vinyl group-containing branched organopolysiloxane (A2) having a branched structure.

### <<Organohydrogen Polysiloxane (B)>>

The silicone rubber-based curable composition of the present embodiment may include a cross-linking agent. It is possible for the cross-linking agent to include an organohydrogen polysiloxane (B) .

The organohydrogen polysiloxanes (B) are classified as linear organohydrogen polysiloxanes (B1) having a linear chain structure and branched organohydrogen polysiloxanes (B2) having a branched structure and it is possible to include either or both.

The insulating silicone rubber-based curable composition and the conductive silicone rubber-based curable composition may include the same type of cross-linking agent. Cross-linking agents of the same type have at least a common structure such as a linear chain structure or branched structure, but may include a molecular weight distribution and different functional groups in the molecule and the addition amounts thereof may be different.

The insulating silicone rubber-based curable composition and the conductive silicone rubber-based curable composition may further include cross-linking agents that differ from each other.

The linear organohydrogen polysiloxane (B1) is a polymer which has a linear chain structure and a structure in which a hydrogen is bonded directly with Si (≡Si-H) and which reacts with the vinyl groups of the components blended in the silicone rubber-based curable composition in addition to the vinyl groups of the vinyl group-containing organopolysiloxane (A) and hydrosilylates and cross-links these components.

The molecular weight of the linear organohydrogen polysiloxane (B1) is not particularly limited, but, for example, the weight average molecular weight is preferably 20000 or less and more preferably equal to or more than 1000 and equal to or less than 10000.

It is possible to measure the weight average molecular weight of the linear organohydrogen polysiloxane (B1), for example, by polystyrene conversion in gel permeation chromatography (GPC) in which chloroform is the expansion solvent.

In addition, usually, the linear organohydrogen polysiloxane (B1) preferably does not have a vinyl group. Due to this, it is possible to precisely prevent the cross-linking reactions from progressing in the molecule of the linear organohydrogen polysiloxane (B1).

As the linear organohydrogen polysiloxane (B1) as described above, for example, an example having the structure represented by Formula (2) is preferably used.

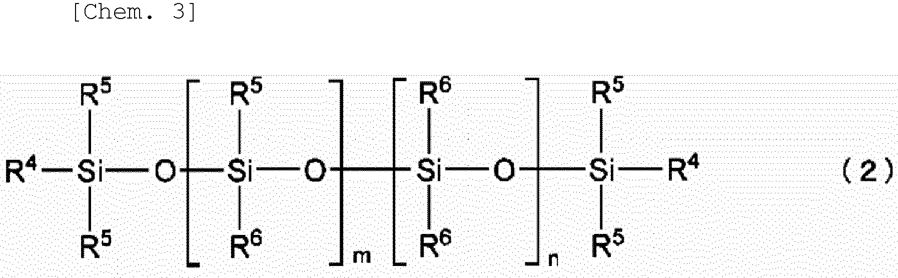

In Formula (2), R⁴ is a substituted or unsubstituted alkyl group, alkenyl group, or aryl group having 1 to 10 carbon atoms, a hydrocarbon group combining these groups, or a hydrido group. Examples of alkyl groups having 1 to 10 carbon atoms include methyl groups, ethyl groups, propyl groups, and the like, among which methyl groups are preferable. Examples of alkenyl groups having 1 to 10 carbon atoms include vinyl groups, allyl groups, butenyl groups, and the like. Examples of aryl groups having 1 to 10 carbon atoms include phenyl groups.

In addition, R⁵ is a substituted or unsubstituted alkyl group, alkenyl group, or aryl group having 1 to 10 carbon atoms, a hydrocarbon group combining these groups or a hydrido group. Examples of alkyl groups having 1 to 10 carbon atoms include methyl groups, ethyl groups, and propyl groups, among which methyl groups are preferable. Examples of alkenyl groups having 1 to 10 carbon atoms include vinyl groups, allyl groups, butenyl groups, and the like. Examples of aryl groups having 1 to 10 carbon atoms include phenyl groups.

In Formula (2), a plurality of R⁴'s are independent of each other and may be different from each other or may be the same. The same also applies to R³. However, at least two or more of the plurality of R⁴'s and R⁵'s are hydrido groups.

In addition, R⁶ is a substituted or unsubstituted alkyl group or aryl group having 1 to 8 carbon atoms, or a hydrocarbon group combining these groups. Examples of alkyl groups having 1 to 8 carbon atoms include methyl groups, ethyl groups, propyl groups, and the like, among which methyl groups are preferable. Examples of aryl groups having 1 to 8 carbon atoms include a phenyl group. The plurality of R⁶'s are independent of each other and may be different from each other or may be the same.

Examples of the substituents of R⁴, R⁵, and R⁶ in Formula (2) include methyl groups, vinyl groups, and the like and methyl groups are preferable from the viewpoint of preventing cross-linking reactions in the molecule.

Furthermore, m and n are the number of repeating units forming the linear organohydrogen polysiloxane (B1) represented by Formula (2), m is an integer of 2 to 150, and n is an integer of 2 to 150. Preferably, m is an integer of 2 to 100 and n is an integer of 2 to 100.

One linear organohydrogen polysiloxane (B1) may be used as one type alone or two or more types may be used in combination.

The branched organohydrogen polysiloxane (B2) is a component that has a branched structure and thus forms regions having a high cross-linking density and contributes significantly to the formation of a structure having a sparse cross-linking density in the silicone rubber. In addition, as in the linear organohydrogen polysiloxane (B1) above, the branched organohydrogen polysiloxane (B2) is a polymer having a structure in which a hydrogen is directly bonded with Si (≡Si-H) and which reacts with the vinyl groups of the components blended in the silicone rubber-based curable composition in addition to the vinyl groups of the vinyl group-containing organopolysiloxane (A) and hydrosilylates and cross-links these components.

In addition, the specific gravity of the branched organohydrogen polysiloxane (B2) is in a range of 0.9 to 0.95.

Furthermore, usually, the branched organohydrogen polysiloxane (B2) preferably does not have a vinyl group. Due to this, it is possible to precisely prevent the cross-linking reactions from progressing in the molecule of the branched organohydrogen polysiloxane (B2).

In addition, as the branched organohydrogen polysiloxane (B2), the example represented by the average composition Formula (c) is preferable.

Average composition Formula (c) (Ha(R⁷)₃₋ₐSiO_{1/2})ₘ(SiO_{4/2})ₙ

(In Formula (c), R⁷ is a monovalent organic group, a is an integer in a range of 1 to 3, m is the number of Hₐ(R⁷)₃₋ₐSiO_{1/2} units, and n is the number of SiO_{4/2} units)

In Formula (c), R⁷ is a monovalent organic group and preferably a substituted or unsubstituted alkyl group or an aryl group having 1 to 10 carbon atoms or a hydrocarbon group combining these groups. Examples of alkyl groups having 1 to 10 carbon atoms include methyl groups, ethyl groups, propyl groups, and the like, among which methyl groups are preferable. Examples of aryl groups having 1 to 10 carbon atoms include phenyl groups.

In Formula (c), a is the number of hydrido groups (hydrogen atoms bonded directly with Si) and is an integer in a range of 1 to 3, preferably 1.

In addition, in Formula (c), m is the number of Hₐ(R⁷)₃₋ₐSiO_{1/2} units and n is the number of SiO_{4/2} units.

The branched organohydrogen polysiloxane (B2) has a branched structure. The linear organohydrogen polysiloxane (B1) and the branched organohydrogen polysiloxane (B2) differ in terms of whether the structures thereof are linear or branched and the number (R/Si) of alkyl groups R bonded with Si when the number of Si is 1 is in a range of 1.8 to 2.1 in the linear organohydrogen polysiloxane (B1) and 0.8 to 1.7 in the branched organohydrogen polysiloxane (B2).

The branched organohydrogen polysiloxane (B2) has a branched structure and thus, for example, the amount of residue is 5% or more when heated to 1000°C in a nitrogen atmosphere at a temperature increase rate of 10°C/min. In contrast, the linear organohydrogen polysiloxane (B1) has a linear structure and thus the amount of residue after heating under the conditions described above is almost zero.

In addition, specific examples of the branched organohydrogen polysiloxane (B2) include an example having the structure represented by Formula (3).

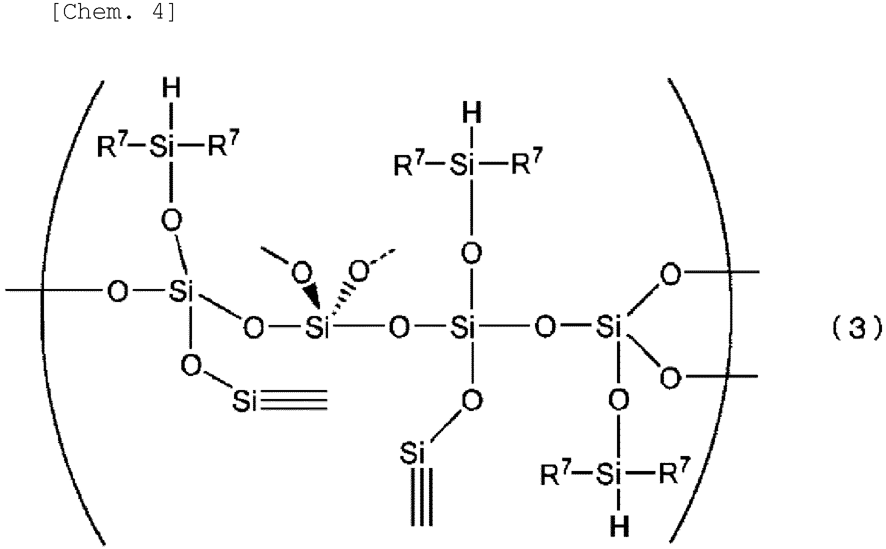

In Formula (3), R⁷ is a substituted or unsubstituted alkyl group or an aryl group having 1 to 8 carbon atoms, or a hydrocarbon group combining these groups, or a hydrogen atom. Examples of alkyl groups having 1 to 8 carbon atoms include methyl groups, ethyl groups, propyl groups, and the like, among which methyl groups are preferable. Examples of aryl groups having 1 to 8 carbon atoms include a phenyl group. Substituents of R⁷ include methyl groups and the like.

In Formula (3), the plurality of R⁷'s are independent of each other and may be different from each other or may be the same.

In addition, in Formula (3), "-O-Si≡" represents that Si has a three-dimensionally spread branched structure.

The branched organohydrogen polysiloxane (B2) may be used as one type alone or two or more types may be used in combination.

In the linear organohydrogen polysiloxane (B1) and the branched organohydrogen polysiloxane (B2), the number of hydrogen atoms (hydrido groups) directly bonded with Si, respectively, is not particularly limited. However, in a silicone rubber-based curable composition, the total amount of hydrido groups in the linear organohydrogen polysiloxane (B1) and the branched organohydrogen polysiloxane (B2) per mole of the vinyl groups in the vinyl group-containing linear organopolysiloxane (A1) is preferably an amount of 0.5 moles to 5 moles and more preferably an amount of 1 mole to 3.5 moles. Due to this, it is possible to reliably form a cross-linked network between the linear organohydrogen polysiloxane (B1) and the branched organohydrogen polysiloxane (B2), and the vinyl group-containing linear organopolysiloxane (A1).

### <<Silica Particles (C)>>

The silicone rubber-based curable composition according to the present embodiment includes a non-conductive filler. The non-conductive filler may include silica particles (C) as necessary. Due to this, it is possible to improve the hardness and mechanical strength of the elastomer.

The insulating silicone rubber-based curable composition and the conductive silicone rubber-based curable composition may include the same type of non-conductive fillers. Non-conductive fillers of the same type have at least a common constituent material and may differ in particle size, specific surface area, surface treatment agent, or the addition amount.

The insulating silicone rubber-based curable composition and the conductive silicone rubber-based curable composition may further include silane coupling agents that differ from each other.

The silica particles (C) are not particularly limited and, for example, fumed silica, calcined silica, precipitated silica, and the like may be used. The above may be used alone or two or more types may be used in combination.

The silica particles (C), for example, preferably have a specific surface area according to the BET method of, for example, 50 m²/g to 400 m²/g and more preferably 100 m²/g to 400 m²/g. In addition, the average primary particle diameter of the silica particles (C) is preferably, for example, 1 nm to 100 nm and more preferably approximately 5 nm to 20 nm.

By using silica particles (C) having a specific surface area and an average particle diameter in such ranges, it is possible to improve the hardness and mechanical strength of the silicone rubber to be formed, in particular, to improve the tensile strength.

### <<Silane Coupling Agent (D)>>

It is possible for the silicone rubber-based curable composition of the present embodiment to include a silane coupling agent (D).

It is possible for the silane coupling agent (D) to have hydrolysable groups. The hydrolysable groups are hydrolyzed by water to become hydroxyl groups and the hydroxyl groups undergo a dehydration-condensation reaction with hydroxyl groups on the surface of the silica particles (C) to modify the surface of the silica particles (C).

The insulating silicone rubber-based curable composition and the conductive silicone rubber-based curable composition may include silane coupling agents of the same type. The silane coupling agents of the same type have at least a common functional group and may differ in other functional groups in the molecule and in the addition amounts.

The insulating silicone rubber-based curable composition and the conductive silicone rubber-based curable composition may further include silane coupling agents that differ from each other.

In addition, it is possible for the silane coupling agent (D) to also include a silane coupling agent having a hydrophobic group. Since this hydrophobic group is imparted to the surface of the silica particles (C) as a result, the aggregation force of the silica particles (C) in the silicone rubber-based curable composition and thus in silicone rubber is decreased (aggregation due to hydrogen bonding due to silanol groups is reduced) and it is assumed that the dispersibility of the silica particles (C) in the silicone rubber-based curable composition is improved as a result. Due to this, the interface between the silica particles (C) and the rubber matrix is increased and the reinforcing effect of the silica particles (C) is increased. Furthermore, it is assumed that the slipperiness of the silica particles (C) in the matrix is improved at the time of rubber matrix deformation. Due to the improved dispersibility and improved slipperiness of the silica particles (C), the mechanical strength of the silicone rubber (for example, tensile strength, tear strength, and the like) is improved by the silica particles (C).

Furthermore, it is possible for the silane coupling agent (D) to include a silane coupling agent having vinyl groups. Due to this, vinyl groups are introduced on the surface of the silica particles (C). Therefore, during curing of the silicone rubber-based curable composition, that is, when the vinyl groups of the vinyl group-containing organopolysiloxane (A) and the hydrido groups of the organohydrogen polysiloxane (B) react to hydrosilylate and form a network (a cross-linking structure), the vinyl groups of the silica particles (C) also contribute to the hydrosilylation reaction with the hydrido groups of the organohydrogen polysiloxane (B) and thus the silica particles (C) are also incorporated into the network. Due to this, it is possible to achieve a lower hardness and a higher modulus in the formed silicone rubber.

As the silane coupling agents (D), it is possible to use silane coupling agents having hydrophobic groups and silane coupling agents having vinyl groups together.

Examples of the silane coupling agents (D) include the agents represented by Formula (4).

Yₙ-Si-(X)₄₋ₙ... (4)

In Formula (4), n represents an integer of 1 to 3. Y represents any functional group having a hydrophobic group, a hydrophilic group or a vinyl group, in which Y is a hydrophobic group when n is 1 and at least one thereof is a hydrophobic group when n is 2 or 3. X represents a hydrolysable group.

The hydrophobic group is an alkyl group or an aryl group having 1 to 6 carbon atoms or a hydrocarbon group combining these groups and examples thereof include a methyl group, an ethyl group, a propyl group, a phenyl group, or the like, among which, a methyl group is particularly preferable.

In addition, examples of the hydrophilic group include a hydroxyl group, a sulfonic acid group, a carboxyl group, a carbonyl group, and the like, among which a hydroxyl group is particularly preferable. The hydrophilic group may be included as a functional group, but is preferably not included from the viewpoint of imparting hydrophobicity to the silane coupling agent (D) .

Furthermore, examples of the hydrolysable groups include alkoxy groups such as methoxy groups and ethoxy groups, chloro groups, silazane groups, or the like, among which silazane groups are preferable due to having a high reactivity with the silica particles (C). Examples having silazane groups as hydrolysable groups are examples having two (Yₙ-Si-) structures in Formula (4) due to the structural characteristics thereof.

Specific examples of the silane coupling agents (D) represented by Formula (4) are as follows.

Examples having a hydrophobic group as a functional group above include alkoxysilanes such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, and decyltrimethoxysilane; chlorosilanes such as methyltrichlorosilane, dimethyldichlorosilane, trimethylchlorosilane, and phenyl trichlorosilane; and hexamethyldisilazane. Among these, silane coupling agents having trimethylsilyl groups, including one or more types selected from the group consisting of hexamethyldisilazane, trimethylchlorosilane, trimethylmethoxysilane and trimethylethoxysilane, are preferable.

Examples having vinyl groups as functional groups as described above include alkoxysilanes such as methacryloxypropyltriethoxysilane, methacryloxypropyltrimethoxysilane, methacryloxypropylmethyldiethoxysilane, methacryloxypropyl methyl dimethoxysilane, vinyltriethoxysilane, vinyltrimethoxysilane and vinylmethyldimethoxysilane; chlorosilanes such as vinyltrichlorosilane and vinylmethyldichlorosilane; and divinyltetramethyldisilazane. Among the above, silane coupling agents having vinyl group-containing organosilyl groups, including one or more selected from the group consisting of methacryloxypropyltriethoxysilane, methacryloxypropyltrimethoxysilane, methacryloxypropylmethyldiethoxysilane, methacryloxypropyl methyl dimethoxysilane, divinyltetramethyldisilazane, vinyltriethoxysilane, vinyltrimethoxysilane, and vinylmethyldimethoxysilane, are preferable.

In addition, in a case where the silane coupling agent (D) includes two types of a silane coupling agent having a trimethylsilyl group and a silane coupling agent having a vinyl group-containing organosilyl group, hexamethyldisilazane as a silane coupling agent having a hydrophobic group and divinyltetramethyldisilazane as a silane coupling agent having a vinyl group are preferably included.

In a case where a silane coupling agent (D1) having a trimethylsilyl group and a silane coupling agent (D2) having a vinyl group-containing organosilyl group are used together, the ratio of (D1) to (D2) is not particularly limited, for example, the weight ratio of (D1): (D2) is 1:0.001 to 1:0.35, preferably 1:0.01 to 1:0.20, and more preferably 1:0.03 to 1:0.15. Using the above numerical ranges makes it possible to obtain the desired silicone rubber properties. Specifically, it is possible to achieve a balance between the dispersibility of silica in the rubber and the cross-linking of the rubber.

In the present embodiment, the lower limit value of the content of the silane coupling agent (D) is preferably 1% by mass or more with respect to 100 parts by weight of the total amount of the vinyl group-containing organopolysiloxane (A), more preferably 3% by mass or more, and even more preferably 5% by mass or more. In addition, the upper limit value of the content of the silane coupling agent (D) is preferably 100% by mass or less with respect to 100 parts by weight of the total amount of vinyl group-containing organopolysiloxane (A), more preferably 80% by mass or less, and even more preferably 40% by mass or less.

By setting the content of the silane coupling agent (D) to the lower limit value above or more, it is possible to improve the adhesiveness between the pillar-shaped portion including the elastomer and the conductive resin layer. In addition, it is also possible to contribute to improving the mechanical strength of the silicone rubber. In addition, by setting the content of the silane coupling agent (D) to the upper limit value above or less, it is possible for the silicone rubber to have appropriate mechanical properties.

### <<Platinum or Platinum Compound (E)>

The silicone rubber-based curable composition according to the present embodiment may include a catalyst. It is possible for the catalyst to include platinum or a platinum compound (E). The platinum or platinum compound (E) is a catalytic component that acts as a catalyst during curing. The addition amount of the platinum or platinum compound (E) is the catalyst amount.

The insulating silicone rubber-based curable composition and the conductive silicone rubber-based curable composition may include the same type of catalyst. The same type of catalyst means having at least a common constituent material, but different compositions may be included in the catalysts and the addition amounts thereof may be different.

The insulating silicone rubber-based curable composition and the conductive silicone rubber-based curable composition may further include different catalysts from each other.

As the platinum or platinum compound (E), it is possible to use known examples, such as examples in which platinum black or platinum is supported on silica, carbon black, or the like, platinic chloride or an alcohol solution of platinic chloride, a complex salt of platinic chloride and an olefin, a complex salt of platinic chloride and vinylsiloxane, and the like.

The platinum or platinum compound (E) may be used as one type alone or two or more types may be used in combination.

In the present embodiment, the content of the platinum or platinum compound (E) in the silicone rubber-based curable composition means the catalyst amount and is able to be set appropriately, but is specifically an amount where the platinum group metal is 0.01 ppm to 1000 ppm as a weight unit with respect to 100 parts by weight of the total amount of the vinyl group-containing organopolysiloxane (A), the silica particles (C), and the silane coupling agent (D), and preferably 0.1 ppm to 500 ppm.

By setting the content of the platinum or platinum compound (E) to the lower limit value above or more, it is possible to cure the silicone rubber-based curable composition at an appropriate rate. In addition, by setting the content of the platinum or platinum compound (E) to the upper limit value above or less, it is possible to contribute to a reduction in production costs.

### <<Water (F)>>

In addition, the silicone rubber-based curable composition according to the present embodiment may also include water (F) in addition to the above components (A) to (E).

The water (F) functions as a dispersant to disperse each component included in the silicone rubber-based curable composition and is a component that contributes to the reaction between the silica particles (C) and the silane coupling agent (D). Therefore, in the silicone rubber, it is possible to more reliably link the silica particles (C) and the silane coupling agent (D) to each other and to exhibit uniform properties as a whole.

### (Other Components)

Furthermore, it is possible for the silicone rubber-based curable composition of the present embodiment to further include other components in addition to the components (A) to (F) described above. Examples of these other components include inorganic fillers other than silica particles (C) such as diatomaceous earth, iron oxide, zinc oxide, titanium oxide, barium oxide, magnesium oxide, cerium oxide, calcium carbonate, magnesium carbonate, zinc carbonate, glass wool, and mica as well as additives such as reaction inhibitors, dispersing agents, pigments, dyes, antistatic agents, antioxidants, flame retardants, and thermal conductivity improvers.

The conductive solution (conductive silicone rubber composition) according to the present embodiment includes the conductive filler and solvent described above in addition to the above-described silicone rubber-based curable composition not including a conductive filler.

It is possible to use various known solvents as the above solvent and to include, for example, high boiling point solvents. The above may be used alone or two or more types may be used in combination.

Examples of the above solvents include aliphatic hydrocarbons such as pentane, hexane, cyclohexane, heptane, methylcyclohexane, ethylcyclohexane, octane, decane, dodecane, and tetradecane; aromatic hydrocarbons such as benzene, toluene, ethyl benzene, xylene, trifluoromethyl benzene, and benzotrifluoride; ethers such as diethyl ether, diisopropyl ether, dibutyl ether, cyclopentyl methyl ether, cyclopentyl ethyl ether, ethylene glycol dimethyl ether, ethylene glycol diethyl ether, diethylene glycol dimethyl ether, 1,4-dioxane, 1,3-dioxane, and tetrahydrofuran; haloalkanes such as dichloromethane, chloroform, 1,1-dichloroethane, 1,2-dichloroethane, 1,1,1-trichloroethane, and 1,1,2-trichloroethane; carbonate amides such as N,N-dimethylformamide and N,N dimethylacetamide; sulfoxides such as dimethylsulfoxide and diethylsulfoxide, and the like. The above may be used alone or two or more types may be used in combination.

By adjusting the amounts of solids or the like in the solution, it is possible for the conductive solution described above to be provided with an appropriate viscosity for various coating methods, such as spray coating and dip coating.

In addition, in a case where the conductive solution includes the conductive filler and the silica particles (C), the lower limit value of the content of the silica particles (C) included in the protrusions 60 is able to be, for example, 1% by mass or more with respect to 100% by mass of the total amount of the silica particles (C) and the conductive filler, preferably 3% by mass or more, and more preferably 5% by mass or more. Due to this, it is possible to improve the mechanical strength of the protrusions 60. On the other hand, the upper limit value of the content of the silica particles (C) included in the protrusions 60 is, for example, 20% by mass or less with respect to 100% by mass of the total amount of the silica particles (C) and the conductive filler, preferably 15% by mass or less, and more, preferably 10% by mass or less. Due to this, it is possible to achieve a balance between conductivity, and mechanical strength and flexibility in the protrusion 60.

Conductive silicone rubber is obtained by heating and drying the conductive solution as necessary.

The conductive silicone rubber may be formed without including silicone oil. Due to this, it is possible to suppress a decrease in conductivity due to the silicone oil bleeding out onto the surface of the protrusions 60.

### <Material of Signal Line 69>

When forming the signal lines 69, it is possible to use known materials, for example, conductive fibers. As conductive fibers, it is possible to use one or more selected from the group consisting of metal fibers, metal-coated fibers, carbon fibers, conductive polymer fibers, conductive polymer-coated fibers, and conductive paste-coated fibers. The above may be used alone or may be used in a combination of two or more.

The metal materials of the above metal fibers and metal-coated fibers are not limited to those having conductivity, but examples thereof include copper, silver, gold, nickel, tin, lead, zinc, bismuth, antimony, stainless steel, aluminum, silver/silver chloride, alloys thereof, and the like. The above may be used alone or may be used in a combination of two or more. Among the above, it is possible to use silver from the point of view of conductivity. In addition, the metal material preferably does not include metals that are harmful to the environment, such as chromium.

The fiber materials of the metal-coated fibers, the conductive polymer-coated fibers, and the conductive paste-coated fibers are not particularly limited, but may be any of synthetic fibers, semi-synthetic fibers, or natural fibers. Among the above, polyester, nylon, polyurethane, silk, cotton, and the like are preferably used. The above may be used alone or may be used in a combination of two or more.

Examples of the carbon fibers include PAN-based carbon fibers, pitch-based carbon fibers, and the like.

For the conductive polymer materials of the conductive polymer fibers and the conductive polymer-coated fibers described above, for example, it is possible to use mixtures of conductive polymers such as polythiophene, polypyrrole, polyaniline, polyacetylene, polyphenylenevinylene, polynaphthalene, and derivatives thereof and binder resins, or aqueous solutions of conductive polymers such as PEDOT-PSS ((3,4-ethylenedioxythiophene)-poly(styrenesulfonic acid)).

The resin material included in the conductive paste of the conductive paste-coated fibers is not particularly limited, but preferably has elasticity and is able to include one or more selected from the group consisting of silicone rubber, urethane rubber, fluoro rubber, nitrile rubber, acrylic rubber, styrene rubber, chloroprene rubber, and ethylene-propylene rubber. The above may be used alone or may be used in a combination of two or more.

The conductive filler included in the conductive paste of the conductive paste-coated fibers is not particularly limited, but known conductive materials may be used and it is possible to include one or more selected from the group consisting of metal particles, metal fibers, metal-coated fibers, carbon black, acetylene black, graphite, carbon fibers, carbon nanotubes, conductive polymers, conductive polymer-coated fibers, and metal nanowires.

The metal forming the conductive filler is not particularly limited, but it is possible to include, for example, at least one of copper, silver, gold, nickel, tin, lead, zinc, bismuth, antimony, silver/silver chloride, or alloys thereof, or two or more of the above. Among these, silver or copper is preferable due to having high conductivity and high availability.

The signal line 69 may be formed of a twisted thread made of a plurality of linear conductive fibers twisted together. Due to this, it is possible to suppress breaking of the signal lines 69 during deformation.

In the present embodiment, for the conductive fibers, coating is not merely covering the outer surface of the fiber material, but, in the case of twisted threads of single fibers twisted together or the like, also includes impregnating a metal, a conductive polymer, or a conductive paste into the inter-fiber gaps in the twisted thread and coating each single fiber forming the twisted thread.

The tensile breaking elongation of the signal line 69 is, for example, equal to or more than 1% and equal to or less than 50% and preferably 1.5% or more to 45%. By setting such numerical ranges, it is possible to suppress excessive deformation of the protrusions 60 while also suppressing breaking during deformation.

### <Material of Electrode Part 80>

The conductive member of the electrode part 80 is, for example, a paste including a good conductive metal. The good conductive metal includes one or more selected from the group consisting of copper, silver, gold, nickel, tin, lead, zinc, bismuth, antimony, or alloys thereof. In particular, silver, silver chloride, and copper are suitable from the viewpoint of availability and conductivity.

In a case of forming the electrode part 80 with a paste including a good-conductive metal, the tops of the protrusions 60 made of a rubber-like elastic body are dipped (dip-coated) into a conductive solution in the form of a paste including a good-conductive metal. Due to this, the electrode part 80 is formed on the electrode formation surface, which is a predetermined region from the tip portions 65 of the protrusions 60 downward.

The electrode part 80, which is a conductive resin layer, may be formed by coating a conductive solution including a conductive filler and a solvent on the electrode formation surface of the protrusions 60. At that time, the adhesiveness of the electrode part 80 (a conductive resin layer) is improved by making the solvent a material (silicone rubber) of the same type as the protrusions 60.

The conductive silicone rubber is obtained by heating and drying the conductive solution as necessary.

The conductive silicone rubber may be formed without including silicone oil. It is possible to suppress a decrease in conductivity due to the silicone oil bleeding out onto the surface of the electrode part 80.

Due to this, it is possible to improve the hair-parting performance when the brain wave measurement device 1 is mounted on the head 99. In addition, it is possible to secure a sufficient contact area for the electrode formation surface (that is, the electrode parts 80) when the brain wave measurement device 1 is mounted.

### <Method for Manufacturing Brain Wave Detection Electrode 50>

It is possible to include the following steps in an example of a method for manufacturing the brain wave detection electrode 50 of the present embodiment.

First, using a mold, the silicone rubber-based curable composition is molded by heating and pressing to obtain a molded body formed of the basal part 51 and the protrusions 60. Subsequently, the signal line 69 is passed through each of the protrusions 60 of the obtained molded body using a sewing needle. A conductive solution in the form of a paste is then dip-coated on the electrode-formation surfaces of the protrusions 60 of the obtained molded body, heated and dried, and then subjected to post-curing. Due to this, it is possible to form the electrode parts 80 on the electrode-formation surfaces of the protrusions 60.

Through the above, it is possible to manufacture the brain wave detection electrode 50.

At the time of the molding steps, insert molding may be used, in which the silicone rubber-based curable composition is introduced into a molding space, in which the signal line 69 is arranged, and molded by heating and pressing.

### <Comparison of Deformation of Protrusions 60>

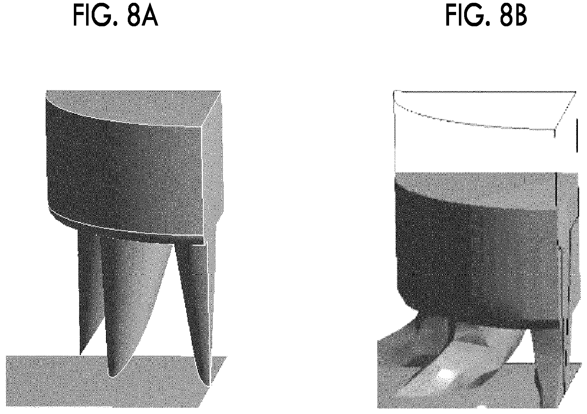

Figs. 7A and 7B and Figs. 8A and 8B show the results of simulating the states of the brain wave detection electrode 50 having the structure of the protrusions 60 of the present embodiment (Figs. 7A and 7B) and a brain wave detection electrode having the structure of a comparison type (Figs. 8A and 8B), which are pressed against the head 99. Here, the deformation state is shown when the protrusions 60 (the electrode part 80) are pressed against a flat plate. Fig. 7A and Fig. 8A show the states before pressing and Fig. 7B and Fig. 8B show the states after pressing. The protrusions of the comparison type have a shape in which the tip portion of a conical shape is cut off at an angle in a plane.

As shown in Fig. 7B, in the structure of the protrusions 60 of the present embodiment, the protrusions 60 deform directly downwards when the protrusions 60 are pressed. That is, the protrusions 60 do not deform in a manner that buckles and bends significantly. As a result, it is possible to suppress slippage of the contact surface between the electrode part 80 and the head 99 and interference with the hair. Meanwhile, as shown in Figs. 8A and 8B, in the case of the structure of the comparison type, as the protrusions (the electrodes) are pressed, slippage may occur on the contact surface between the electrode parts and the scalp and the hair may interfere with the electrodes during the process.

### <Summary of Features of Brain Wave Detection Electrode 50>

The features of the brain wave detection electrode 50, in particular, the structure of the protrusion 60, will be summarized and explained.
(1) The brain wave detection electrode 50 includes
   the pillar-shaped basal part 51 (base part);
   a plurality of the polygonal pyramid-shaped protrusions 60 (protruding parts) that are elastic bodies and provided perpendicularly to one end face (the protrusion formation surface 52) of the basal part 51; and
   the electrode parts 80 provided on the tip portions of the protrusions 60,
   in which H/D, which is a ratio As of the width D of the bottom surface of the polygonal pyramid shapes to the height H of the polygonal pyramid shapes, is equal to or more than 1.2 and equal to or less than 2.0.

By making the protrusions 60 polygonal pyramids and setting the ratio As to the height H to such a range, when the brain wave detection electrodes 50 are pressed against the head 99, it is possible to prevent deformation such as buckling of the protrusions 60 despite a small pressing force and to realize an appropriate contact state.

(2) The polygonal pyramid shapes are square pyramids.

In a case where the polygonal pyramid shapes presented by the protrusions 60 are square pyramids, it is easy to design the arrangement of the plurality of the protrusions 60 and also to predict the deformation state when pressed against the head 99, resulting in a stable brain wave reading.

(3) In the plurality of the protrusions 60, the polygons of the bottom surfaces of adjacent protrusions 60 are in contact with each other.

Due to this, it is possible to arrange a large number of the protrusions 60 on a protrusion body 61 (the electrode formation surface 62) and also to suppress excessive deformation of the protrusions 60.

(4) The number N of the protrusions 60 is equal to or more than 4 and equal to or less than 30.

By setting the number N of the protrusions 60 to the above range, it is possible to appropriately adjust the state of contact with the head 99.

(5) The shortest distance L between the vertices (the tip portions 65) of each of the adjacent protrusions 60 is equal to or more than 2 mm and equal to or less than 5 mm.

By setting the shortest distance L between the tip portions 65 to such a range, it is possible to carry out the brain wave measurement by bringing the electrode parts 80 into contact at the optimum contact positions. In addition, it is possible to part the hair smoothly and to prevent hair from getting between the electrode parts 80.

(6) The hardness E of the elastic bodies (that is, the protrusions 60) is equal to or more than 15 and equal to or less than 55.

By setting the hardness E in such a range, it is possible to achieve a balance between the deformation resistance and the suppression of discomfort, pain, and the like when the brain wave detection electrodes 50 are pressed.
(7) The protrusions 60 and the basal part 51 are formed integrally.
(8) The brain wave measurement device 1 is provided with the brain wave detection electrodes 50 described above.

Embodiments of the present invention were described above with reference to the drawings, but these are examples of the invention and it is also possible to adopt various configurations (modification examples) other than those described above. Examples

A detailed description will be given of the present invention with reference to Examples, but the present invention is not limited in any way to the description of these Examples.

In the following examples, as shown in Table 1, twelve brain wave detection electrodes 50 having different shapes were created using a 3D printer and evaluated for (1) deformation behavior, (2) hair-parting property, and (3) pain in a short time and contact feeling. Shapes 1 and 2 in Table 1 correspond to Comparative Examples 1 and 2 and shapes 3 to 8 correspond to Examples 1 to 6. For all of the shapes, the material of the brain wave detection electrodes 50 had a hardness of 27.

### (1) Deformation Behavior

Deformation behavior (whether buckling does not occur) was evaluated by observing how the protrusions 60 of the brain wave detection electrode were deformed and whether or not buckling occurred easily when the protrusions were pressed against a transparent plate with a plurality of forces (here, 0 N, 2 N, 4 N, 6 N and 8 N). The evaluation results are indicated by "o", "△" and "×" in the table. The criteria for the above are as follows.
∘: No buckling deformation even at high loads (6 N or more).
△: No buckling at low loads (4 N or less), but buckling at high loads (6 N or more).
×: Buckling at low loads (4 N or less).

The evaluation results confirmed a tendency in which, the higher the ratio As is, the more likely it is for buckling deformation to occur, even at low loads.

### (2) Hair-Parting Property

For the evaluation of the hair-parting property, simulated hair was placed over a transparent plate and evaluation was carried out by observing whether or not the simulated hair interfered with the region corresponding to the electrode parts 80 of the protrusions 60. The evaluation results are indicated by "∘", "△" and "×" in the table. The criteria for the above are as follows.
∘: Half or more of the protrusions 60 parted the hair and came in contact with the scalp.
△: Less than half of the protrusions 60 parted the hair and came in contact with the scalp.
×: All of the protrusions 60 ended up over the hair and did not come in contact with the scalp.

In Examples 1 to 3 (shapes 3 to 5), the hair-parting property was good.

The shapes having a smaller outer diameter at the base part (the basal part 51) had slightly better hair-parting performance. This is considered to be because hair tends to flow to the outside of the protrusions 60 (the electrode parts 80) when the outer diameter of the base part is small. However, it is considered that, when the width D of the protrusions is small, the number N of the protrusions 60 in the same area becomes larger, the spacings between the protrusions 60 become narrower, and the number of spacings increases, thus, the hair gets stuck in these spacings and the outside-flowing performance is decreased.

### (3) Pain in Short Time and Contact Feeling

For shape 1 and shapes 3 to 8 (Comparative Example 1 and Examples 1 to 6), for which the evaluation result was "no buckling" in the evaluation of deformation behavior, it was evaluated whether pain in a short time or a contact feeling was felt. The evaluation results are indicated by "∘", "△" and "×" in the table. The criteria for the above are as follows. First, sensory evaluation was carried out for the contact feeling using the following four levels when the protrusions 60 (the electrode parts 80) were pressed on the top of the heads of a plurality of persons for one minute with a load of 4 N.
1: There is a contact feeling but it is not bothersome.
2: No pain is felt, but there is a feeling of discomfort.
3: Pain is felt, but it is tolerable for a short time.
4: Pain is felt and it is unbearable even for a short time.

The above are shown in the table as "o", "△", and "×" according to the average value of the sensory evaluation.
∘: Less than 1.5
△: 1.5 or more and less than 2.5
×: 2.5 or more.

In the overall evaluations of (1) to (3), shape 3 and shape 4 (Example 1 and Example 2) were the best.

### [Table 1]

**Table 1**

| | Comparative Example 1 | Comparative Example 2 | Example 1 | Example 2 | Example 3 | Example 4 | Example 5 | Example 6 |
|---|---|---|---|---|---|---|---|---|
| Shape No. | (1) | (2) | (3) | (4) | (5) | (6) | (7) | (8) |
| Lattice number [-] N | 21 | 21 | 12 | 7 | 4 | 24 | 13 | 8 |
| Height [mm] H | 2.2 | 6 | 4 | 5 | 6 | 4 | 5 | 6 |
| Width [mm] D | 2.2 | 2.2 | 2.9 | 3.6 | 4.5 | 2.9 | 3.7 | 4.5 |
| Aspect ratio As | 1 | 2.73 | 1.38 | 1.39 | 1.33 | 1.38 | 1.35 | 1.33 |
| Base diameter [mm] Φ | 14 | 14 | 14 | 14 | 14 | 20 | 20 | 20 |
| Shape | 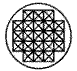 | 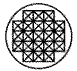 | 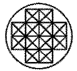 | 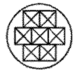 | 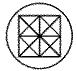 | 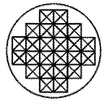 | 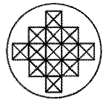 | 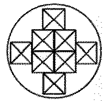 |
| Deformation behavior (does buckling occur?) | ○ | × | ○ | ○ | ○ | ○ | ○ | ○ |
| Hair-parting property | × | × | ○ | ○ | ○ | △ | △ | △ |
| Pain in short time/Contact feeling | ○ | - | ○ | ○ | △ | △ | △ | △ |

This application claims priority based on Japanese Patent Application No. 2021-032537 filed March 2, 2021.

### REFERENCE SIGNS LIST

- 1: Brain wave measurement device
- 10: Brain wave electrode unit
- 20: Frame
- 21: Electrode unit mounting section
- 50: Brain wave detection electrode
- 51: Basal part
- 52: Protrusion formation surface
- 54: Side surface
- 60: Protrusion
- 61: Protrusion body
- 63: Protrusion bottom surface
- 65: Tip portion
- 80: Electrode part

## Claims

1. A brain wave detection electrode (50) comprising:
a pillar-shaped base part (51);
a plurality of polygonal pyramid-shaped protruding parts (60) that are elastic bodies and provided perpendicularly to one end face of the base part (51); and
electrode parts (80) provided on tip portions (65) of the protruding parts (60),
wherein H/D, which is a ratio As of a width D of a bottom surface of the polygonal pyramid shapes to a height H of the polygonal pyramid shapes, is equal to or more than 1.2 and equal to or less than 2.0,
wherein the polygonal pyramid shape is a square pyramid or a hexagonal pyramid,
wherein, in the plurality of the protruding parts (60), polygons of bottom surfaces of adjacent protruding parts (60) are in contact with each other,
wherein a shortest distance L between vertices (65) of adjacent protruding parts (60) is equal to or more than 2 mm and equal to or less than 5 mm, and
wherein a type A durometer hardness E measured at 37°C and in accordance with JIS K 6253 (1997) of the elastic bodies is equal to or more than 15 and equal to or less than 55.

2. The brain wave detection electrode (50) according to claim 1, wherein a number N of the protruding parts (60) is equal to or more than 4 and equal to or less than 30.

3. The brain wave detection electrode (50) according to claim 1 or 2, wherein the protruding parts (60) and the base part (51) are formed integrally.

4. A brain wave measurement device (1) comprising the brain wave detection electrode (50) according to any one of claims 1 to 3.

## Patentansprüche

1. Elektrode zur Erfassung von Gehirnwellen (50), umfassend:
einen säulenförmigen Basisteil (51);
eine Vielzahl von polygonalen, pyramidenförmigen Vorsprüngen (60), die elastische Körper sind und senkrecht zu einer Endfläche des Basisteils (51) angeordnet sind; und
Elektrodenabschnitte (80), die an den Spitzenabschnitten (65) der Vorsprünge (60) vorgesehen sind,
wobei H/D, das ein Verhältnis As der Breite D einer Grundfläche der polygonalen Pyramidenformen zur Höhe H der polygonalen Pyramidenformen ist, gleich oder größer als 1,2 und gleich oder kleiner als 2,0 ist,
wobei die polygonale Pyramidenform eine quadratische Pyramide oder eine sechseckige Pyramide ist,
wobei bei der Vielzahl der Vorsprünge (60) die Polygone der Unterseiten benachbarter Vorsprünge (60) miteinander in Kontakt stehen,
wobei ein kürzester Abstand L zwischen den Scheitelpunkten (65) benachbarter Vorsprünge (60) 2 mm oder mehr und 5 mm oder weniger beträgt, und
wobei die Durometer-Typ A-Härte E der elastischen Körper, gemessen bei 37°C und gemäß JIS K 6253 (1997), 15 oder mehr und 55 oder weniger beträgt.

2. Elektrode zur Erfassung von Gehirnwellen (50) gemäß Anspruch 1, wobei die Anzahl N der Vorsprünge (60) gleich oder größer als 4 und gleich oder kleiner als 30 ist.

3. Elektrode zur Erfassung von Gehirnwellen (50) nach Anspruch 1 oder 2, wobei die Vorsprünge (60) und der Basisteil (51) einstückig ausgebildet sind.

4. Vorrichtung zur Messung von Gehirnwellen (1), umfassend die Elektrode zur Erfassung von Gehirnwellen (50) nach einem der Ansprüche 1 bis 3.

## Revendications

1. Électrode de détection d'ondes cérébrales (50) comportant :
une partie de base en forme de colonnette (51) ;
une pluralité de parties saillantes (60) en forme de pyramides polygonales qui sont des corps élastiques et sont disposées perpendiculairement à une face d'extrémité de la partie de base (51) ; et
des parties d'électrode (80) disposées sur les parties de pointe (65) des parties saillantes (60),
dans laquelle H/D, qui est un rapport As d'une largeur D d'une surface inférieure des formes de pyramide polygonale sur une hauteur H des formes de pyramide polygonale, est égal ou supérieur à 1,2 et égal ou inférieur à 2,0,
dans laquelle la forme de pyramide polygonale est une pyramide carrée ou une pyramide hexagonale,
dans laquelle, dans la pluralité des parties saillantes (60), des polygones de surfaces inférieures de parties saillantes (60) adjacentes sont en contact les uns avec les autres,
dans laquelle une distance la plus courte L entre des sommets (65) de parties saillantes (60) adjacentes est égale ou supérieure à 2 mm et égale ou inférieure à 5 mm, et
dans laquelle une dureté E au duromètre de type A, mesurée à 37°C et conformément à la norme JIS K 6253 (1997) des corps élastiques est égale ou supérieure à 15 et égale ou inférieure à 55.

2. Électrode de détection d'ondes cérébrales (50) selon la revendication 1, dans laquelle un nombre N des parties saillantes (60) est égal ou supérieur à 4 et égal ou inférieur à 30.

3. Électrode de détection d'ondes cérébrales (50) selon la revendication 1 ou 2, dans laquelle les parties saillantes (60) et la partie de base (51) sont formées d'un seul tenant.

4. Dispositif de mesure d'ondes cérébrales (1) comportant l'électrode de détection d'ondes cérébrales (50) selon l'une quelconque des revendications 1 à 3.
